(19) Europäisches Patentamt
European Patent Office
Office européen des brevets

(11) **EP 1 729 778 B1**

(12) **EUROPÄISCHE PATENTSCHRIFT**

(45) Veröffentlichungstag und Bekanntmachung des Hinweises auf die Patenterteilung:
**11.06.2008 Patentblatt 2008/24**

(21) Anmeldenummer: 05716250.5

(22) Anmeldetag: **19.03.2005**

(51) Int Cl.:
*A61K 31/585* (2006.01)       *A61K 47/32* (2006.01)

(86) Internationale Anmeldenummer:
**PCT/EP2005/002971**

(87) Internationale Veröffentlichungsnummer:
**WO 2005/097196 (20.10.2005 Gazette 2005/42)**

(54) **PHARMAZEUTISCHE ZUBEREITUNG ZUR APPLIKATION AUF DIE HAUT MIT EINEM GEHALT AN DROSPIRENON**

PHARMACEUTICAL PREPARATION CONTAINING DROSPIRENONE FOR APPLICATION TO THE SKIN

PREPARATION PHARMACEUTIQUE CONTENANT DE LA DROSPIRENONE, DESTINEE A ETRE APPLIQUEE SUR LA PEAU

(84) Benannte Vertragsstaaten:
**AT BE BG CH CY CZ DE DK EE ES FI FR GB GR HU IE IS IT LI LT LU MC NL PL PT RO SE SI SK TR**
Benannte Erstreckungsstaaten:
**AL BA HR LV MK YU**

(30) Priorität: **01.04.2004 DE 102004016779**

(43) Veröffentlichungstag der Anmeldung:
**13.12.2006 Patentblatt 2006/50**

(73) Patentinhaber: **Bayer Schering Pharma Aktiengesellschaft**
**13353 Berlin (DE)**

(72) Erfinder: **BRACHT, Stefan**
**16548 Glienicke Nordbahn (DE)**

(56) Entgegenhaltungen:
**EP-A- 0 267 051          US-A- 5 352 457**
**US-B1- 6 177 416**

**Beschreibung**

**Technisches Gebiet**

Die Erfindung betrifft eine pharmazeutische Zubereitung zur Applikation auf die Haut mit einem Gehalt an Drospirenon. Dabei liegt die in der Zubereitung enthaltene Menge Drospirenon in deren Ausgangszustand nicht oberhalb der Sättigungslöslichkeit, während die Sättigungslöslichkeit des Drospirenons nach dem Auftragen der Zubereitung auf die Haut durch Austritt von lösungsvermittelnden Komponenten überschritten wird.

**Stand der Technik**

Steroidhormone werden heute bereits in einer großen Anzahl transdermal verabreicht. Dies geschieht entweder im Rahmen der Hormonersatztherapie bei Mann und Frau oder aber zur Empfängnisverhütung bei der Frau.

Trotz der Vielzahl der transdermalen Arzneiformen handelt es sich bisher nur um eine kleine Anzahl von steroidalen Wirkstoffen, die auf diesem Wege verabreicht werden: Aus der Gruppe der Estrogene betrifft dies Estradiol und Ethinylestradiol (z.B. Estraderm, Climara®, Fem7, OrthoEVRA®, EstraDOT), bei den Gestagenen sind es Levonorgestrel, Norethisteron und Norethisteronacetat (z.B. Fem7 Combi, CombiPatch) sowie Norelgestromin .

In der Andrologie steht Testosteron in transdermalen Arzneiformen zur Verfügung (z.B. Androderm®, Testoderm®, Testogel®).

Die Machbarkeit der transdermalen Anwendung beruht jedoch nicht auf der guten Hautgängigkeit der Steroidhormone, sondern allein auf Ihrer hohen Wirkpotenz. Ethinylestradiol und Levonorgestrel entfalten ihre Wirkung bereits bei Tagesdosen im Bereich von 20 bis 50 $\mu$g pro Tag. Norethisteronacetat gehört mit einer typischen transdermalen Tagesdosis von 125-250 $\mu$g/d bereits zu den schwächer wirksamen Stoffen.

Unter den heute therapeutisch verwendeten Gestagenen zählen Dienogest und Drospirenon bezogen auf die erforderliche Tagesdosis zu den weniger potenten Substanzen, da sie im Bereich typischer oraler Tagesdosen von 2 bis 3 mg verabreicht werden müssen, um beispielsweise ihre kontrazeptive Wirkung zu entfalten. Im Gegensatz zu so genannten hochpotenten Gestagenen wie z.B. Gestoden oder Norelgestromin, deren Tagesdosis im unteren, zweistelligen Mikrogrammbereich liegt, mussten Drospirenon und Dienogest bisher als ungeeignet für die transdermale Verabreichung angesehen werden.

Das einzige Steroidhormon, das bisher transdermal mit Tagesdosen im Milligrammbereich verabreicht werden kann, ist Testosteron. Allerdings sind die kommerziellen Pflasterprodukte Testoderm® und Androderm® für ihre geringe lokale Verträglichkeit bzw. die geringe Patientenakzeptanz wegen des Ortes der Anwendungs - am Hodensack (Testoderm®) - bzw. der erforderlichen Pflastergröße von 74 cm$^2$ (Androderm®, 2 Pflaster gleichzeitig appliziert) bekannt.

Andererseits ist für die transdermale Testosterontherapie das Gelpräparat Testogel® verfügbar, das ebenfalls die Resorption von etwa 5-10 mg Testosteron pro Tag bei einem geringen lokalen Irritationspotential und verbessertem Anwendungskomfort erlaubt.

Aus der Patentliteratur sind eine große Anzahl von pharmazeutischen Zubereitungen zur Applikation auf die Haut mit einem Gestagengehalt bekannt. Die US-Schrift 5352457 beschreibt transdermale System zur Verabreichung von Progesteron.

Die WO-Schrift 97/11680 A1 beschreibt einen Spender zur Verabreichung von Gestagenen und Estrogenen.

Die DE-Patentschrift 199 06 152 A1 beansprucht eingangs Reservoirsysteme, in welchen der Wirkstoff in leicht flüchtigen Lösungsmitteln, wie Ethanol gelöst ist. Es wird die transdermale Verabreichung von Wirkstoffen mittels adhäsiven Matrixsystem beschrieben.

die WO-Schrift 99/15156 A1 beschreibt ein Verfahren zur Herstellung einer transdermalen Verabreichungsform, wobei der/die Wirkstoff/e steroidale Hormone sein können und diese in der Matrix auf dem Träger im gesättigten oder übersättigten Zustand vorliegen.

**Darstellung der Erfindung**

Aufgabe der Erfindung ist es, transdermale Verabreichungsformen für die Applikation auf die Haut für weniger potente Gestagene zu finden.

Erfindungsgemäß wird die Aufgabe durch eine pharmazeutische Zubereitung zur Applikation auf die Haut mit einem Gehalt an Drospirenon gelöst, bei welchem die enthaltene Menge Drospirenon in der Zubereitung in deren Ausgangszustand nicht oberhalb der Sättiaunaslöslichkeit liegt und die Sättigungslöslichkeit des Drospirenons nach dem Auftragen der Zubereitung auf die Haut durch Austritt von lösungsvermittelnden Komponenten aus der Zubereitung überschritten wird.

Erfindungsgemäß kann Sättigungslöslichkeit des Drospirenons in der Zubereitung während der Applikation um mindestens den Faktor 5, vorzugsweise um mindestens den Faktor 10 überschritten werden.

Auch kann die Sättigungslöslichkeit des Drospirenons in der Zubereitung während der Applikation nach 1 Stunde bereits um mindestens den Faktor 2, vorzugsweise um mindestens den Faktor 5 überschritten werden.

Die pharmazeutische Zubereitung kann einen Gehalt an Ethanol oder Isopropanol aufweisen.

Erfindungsgemäß kann die pharmazeutische Zubereitung bezogen auf Drospirenon ein kristallisationshemmender Zusatz und/oder einen Gehalt an mindestens einem Permeationsbeschleuniger enthalten.

Es wurde gefunden, dass es sich bei dem kristallisationshemmenden Zusatz um ein lösliches Polyvinylpyrrolidon handelt.

Die pharmazeutische Zubereitung kann eine halbfeste Zubereitung in der Form eines alkoholhaltigen Gels oder eine flüssige, alkoholhaltige Zubereitung in der Form einer Lotion, eines Schaums oder eines Sprays sein.

Auch kann es sich bei der flüssigen oder halbfesten, alkoholhaltige, Zubereitung um einen Bestandteil eines transdermalen Pflasters vom Typ eines Reservoirsystems handeln.

Es wurde weiterhin gefunden, dass die Sättigungslöslichkeit des Drospirenons in der Zubereitung während der Applikation um mindestens den Faktor 1,5 , vorzugsweise um mindestens den Faktor 2 überschritten wird und dass die alkoholische Zubereitung einen Alkoholgehalt von mindestens 60% (m/m), vorzugsweise von mindestens 65% (m/m) aufweist.

Neben Drospirenon kann auch ein Estrogen enthalten sein.

Auch wurde gefunden, dass das enthaltene Estrogen während der Applikation auf der Haut ebenfalls vom untersättigten oder gerade gesättigten Zustand in den übersättigten Zustand übergeht.

Das Estrogen kann Ethinylestradiol sein.

Bei der besonders bevorzugten Ausführungsform der Erfindung handelt es sich um ein transdermales Gel mit einem Gehalt an Ethanol und Wasser. Der Wirkstoff Drospirenon liegt vollständig gelöst vor. Um eine ausreichende Löslichkeit von Drospierenon mit Gehalten von bevorzugt 0,2 -2 % (m/m), besonders bevorzugt 0,5 bis 1 % (m/m) in dem Gel zu gewährleisten, wird weiterhin ein Ethanolgehalt von mindestens 60% (m/m), besonders bevorzugt sogar mindestens 65% (m/m) vorgesehen.

Wegen des hohen Alkoholgehaltes kommen neben den ionischen Gelbildnern auf Poylacrylsäurebasis (Carbomere oder Carbopole, z.B. Carbopol 940,941 oder 980) auch Gelbildner aus der Gruppe von Cellulosederivaten in Betracht, beispielsweise Hydroxymethyl-, Hydroxyethylcellulose oder Hydroxypropylcellulose.

Als weitere Zusatzstoffe kommen vor allem Permeationsbeschleuniger, Rückfettungsmittel und Antioxidantien in Betracht.

Um die Übersättigung der erfindungsgemäßen Systeme an Drospirenon während der Anwendung zu ermöglichen, wird der Einsatz einer Gruppe von niedermolekularen Hilfsstoffen bevorzugt, die einerseits ein gutes Lösungsvermögen für Drospirenon aufweisen, weiterhin entweder durch hohe Flüchtigkeit oder aber durch besonders gute Hautgängigkeit unter den Bedingungen der Anwendung aus der Zubereitung entweichen, wodurch eine Übersättigung an Drospirenon realisiert wird. Diese bevorzugten Hilfsstoffe weisen abschließend auch eine gute toxikologische Verträglichkeit bei der Anwendung auf der Haut auf.

Solche Stoffe werden bevorzugt aber nicht ausschließlich ausgewählt aus der Gruppe von Ethanol, Isopropanol (2-Propanol), 1,2-Propandiol (Propylenglykol), Dipropylenglykol, 1,3-Butandiol, Dieethylenglykolmonomethyl und -ethylether, Propylencarbonat und Isopropylidenglycerol sowie Monoterpenbestandteilen von ätherischen Ölen.

Diese aus der Formulierung durch Verdunstung oder transdermale Absorption leicht entweichenden Lösungsvermittler für Drospirenon können nach Bedarf mit einem oder mehreren Co-solventien kombiniert werden, die weder leicht verdampfbar noch gut dermal absorbierbar sind.

Eine weiterhin bevorzugte Ausführungsform der vorliegenden Erfindung sind transdermale Systeme vom Reservoirtyp.

Für die enthaltenen halbfeste Zubereitung gelten die gleichen Ausführungen wie für transdermale Gele. Die Herstellung von Reservoirsystemen und Ihre Befüllung kann nach bekannter Weise erfolgen.

Es wurde festgestellt, dass Drospirenon bei der Applikation in einem hydro-ethanolischen Vehikel durch Maushaut in vitro nahezu ebenso gut permeiert wie Testosteron. Hieraus kann geschlossen werden, dass auch bei der Applikation am Menschen Tagesdosen von Drospirenon im unteren Milligrammbereich ähnlich wie bei Testosteron erzielt werden können. Dieser unerwartete Befund steht vermutlich mit einem sehr hohen Maß der Übersättigung des Wirkstoffes während der Eintrocknung des Vehikels bei der Anwendung in Verbindung. Zwar wird der Mechanismus der Übersättigung durch Verdunstung insbesondere des Alkoholanteiles aus transdermalen Gelen, Lotionen oder Sprays diskutiert, jedoch haben sich bei den erfindungsgemäßen Formulierungen unerwartet hohe Übersättigungsgrade für Drospirenon als zweckmäßig und praktikabel erwiesen.

Bestimmung des Übersättigungsfaktors, der während der Applikation der betreffenden Zubereitung auftritt:

- Bei halbfesten Gelen, Lotionen, Schäumen und Sprays:

Zur Vereinfachung der Untersuchungen und insbesondere der analytischen Probenverarbeitung werden die Bestimmungen der Löslichkeit von Drospirenon in diesen Zubereitungen bzw. den daraus durch Eintrocknung entstehenden Resten auf halbtheoretischer Basis vorgenommen . Der halbtheoretische Ansatz besteht darin, dass für diese Betrachtung nur die flüssigen Bestandteile der Formulierung herangezogen werden. Im Falle von Gelen, Lotionen und Sprays ist der Anteil des gel- bzw. Film bildenden oder verdickenden Polymers in der Regel mengenmäßig vernachlässigbar, stört aber häufig die analytische Bearbeitung der Proben erheblich. Eine Prüflösung aus allein den flüssigen Bestandteilen wird im Rahmen dieser Erfindung stellvertretend für die genannten Zubereitungen herangezogen. Im konkreten Fall der Formulierung aus Beispiel 2 wird stellvertretend folgende Mischung untersucht:

- Prüflösungsbeispiel (vor der Eintrocknung)

| | Teile (m/m) |
|---|---|
| Isopropylmyristat | 1,00 |
| gereinigtes Wasser | 23,55* |
| Ethanol 96% | 73,50 |
| *Summe aus 20,0 g Wasser zzgl. 3,55 g Wasser aus 3,70 g 0,1 N NaOH (NaOH wird nicht verwendet, da dies nur in Anwesenheit des Polymers sinnvoll ist, in dessen Abwesenheit aber ein stark alkalisches, zersetzendes Milieu schaffen würde). | |

Die Bestimmung der Löslichkeit von Drospirenon in der Prüflösung vor der Eintrocknung wird wie folgt durchgeführt:

Etwa 10 g gewogene Prüflösung werden unter Rühren mit einem Magnetrührfisch solange mit Drospirenon versetzt, bis sich ein deutlich sichtbarer Bodensatz bildet. Diese Lösung wird mindestens 24 Stunden lang gerührt, wonach der Bodensatz weiterhin vorhanden sein muss. Andernfalls wird weiteres Drospirenon zugesetzt und der Vorgang wird wiederholt. Nach Beendigung des Rührvorganges wird ca. 1,0 ml Prüflösung entnommen und in ein 1,5 ml Autosamplergefäß mit Schraubdeckel überführt. Die Probe wird abschließend 10 Minuten bei mindestens 3000 G zentrifugiert, um das ungelöste Drospirenon am Boden der Probe abzuscheiden. In einem Aliquot des erhaltenen Überstandes wird sodann die Konzentration des Drospirenons mit einer geeigneten HPLC-Methode bestimmt. Der ermittelte Wert entspricht der Sättigungslöslichkeit von Drospirenon in der Testmischung vor der Eintrocknung (Drospirenon$_{S1}$ [mg/ml]).

Für erfindungsgemäße Zubereitungen gilt die Bedingung Drospirenon$_{Init}$ kleiner/gleich Drospirenon$_{S1}$, wobei Drospirenon$_{Init}$ [mg/ml] die Ausgangskonzentration an Drospirenon in der Zubereitung ist.

- Die Bestimmung der Löslichkeit von Drospirenon in der flüssigen Testmischung nach der Eintrocknung wird wie folgt durchgeführt:

Die Prüflösung wird mit einer Schichtdicke von etwa 50 $\mu$m auf einem flächigen, inerten Träger ausgebreitet und für einen definierten Zeitraum bei ca. 32°C (Hautoberflächentemperatur) unter Raumbedingungen ohne Abdeckung eingetrocknet.

Der Zeitraum richtet sich nach der vorgesehenen Anwendungsdauer; bei einer einmal täglichen Applikation entspricht er 24 Stunden. Es kann auch ein kürzerer Zeitraum gewählt werden, wenn beispielsweise der Grad der Eintrocknung zu einem früheren Zeitraum als dem Applikationsende ermittelt werden soll.

Die Schichtdicke von 50 $\mu$m wird für den Geltungsbereich dieser Erfindung als Standardschichtdicke für die transdermale Applikation von Gelen, Lotionen oder Sprays definiert. Im Falle des kommerziellen Testogels werden z.B. 5 ml Gel auf den Oberarm aufgetragen. Bei der hier definierten Standardschichtdicke von 50 $\mu$m ergibt sich für 5 ml eine theoretische Applikationsfläche von etwa 90 cm$^2$ (angenommene Dichte des Gels ca. 0,9 g/cm$^3$) wodurch eine ausreichende Näherung an die Applikation in vivo erreicht wird.

Im Falle des obigen Prüflösungsbeispiels werden etwa 4,36 g Prüflösung, genau gewogen (Gewicht = G$_{P1}$), in einer tarierten Petrischale mit 10 cm Durchmesser (entspr. 78,53 cm$^2$) ausgebreitet, wobei sich eine Schichtdicke von etwa 50 $\mu$m ergibt.

Auf einer Wärmeplatte wird dieser Ansatz bei ca. 32°C über einen Zeitraum von 24 Stunden (entsprechend einmal täglicher Applikation) eingetrocknet. Die Menge der Prüflösung und der Durchmesser der Schale sind beispielhaft und nicht limitierend. Die Werte können innerhalb des geschilderten Zusammenhangs variiert werden. Die Testmischung wird während der Prüfung nicht mechanisch bewegt oder gemischt, sondern ruhig stehen gelassen. Es wird keine besondere Luftzirkulation über der Probe vorgenommen.

Am Ende des Versuchs wird das Gewicht des eingetrockneten Restes der Prüflösung ermittelt (Gewicht = $G_{P2}$).

Aus den erhaltenen Werten wird der Eintrocknungsfaktor der Prüflösung nach Gleichung 1 errechnet:

$$F_E = G_{P1} / G_{P2}$$

Von der Prüflösung in der Zusammensetzung vor der Eintrocknung werden etwa 250,0 g, genau gewogen (Gewicht= $G_{R1}$) in einen tarierten 500 ml Weithalskolben vorgelegt.

Anschließend wird dieser Ansatz in einem Rotavapor bei 32°C unter Unterdruck eingeengt, bis die verbleibende Menge der Zubereitung zu der Ausgangsmenge in dem selben Gewichtsverhältnis steht, wie es nach der Eintrocknung in einer Petrischale über einen definierten Zeitraum gefunden wurde, bis also die im Kolben verbliebene Gewichtsmenge (Gewicht = $G_{R2}$) die Gleichung 2 erfüllt:

$$G_{R2} = G_{R1} / F_E$$

Der vergrößerte Ansatz der Prüflösung im Rotavapor dient der Gewinnung einer ausreichend großen Rückstandsmenge für die anschließende Bestimmung der Löslichkeit von Drospirenon. Es wird wiederum bei 32°C gearbeitet, um die Verdunstungsverhältnisse auf der Hautoberfläche in quantitativ beschleunigter aber qualitativ möglichst wenig verfälschter Form zu reproduzieren.

Von der schließlich erhaltenen Rückstandsmenge im Kolben wird 1,0 g* entnommen und in ein 1,5 ml Autosamplergefäß mit Schraubdeckel überführt. Diesem Rückstand werden 100 mg Drospirenon zugesetzt. Anschließend wird dieser Ansatz eine Stunde lang in einem Ultraschallbad behandelt. Danach bleibt die Probe 24 Stunden lang unter Raumbedingungen stehen. Die Probe wird abschließend 10 Minuten bei mindestens 3000 G zentrifugiert, um das ungelöste Drospirenon am Boden der Probe abzuscheiden. In einem Aliquot des erhaltenen Überstandes wird sodann die Konzentrations des Drospirenons mit einer geeigneten HPLC-Methode bestimmt. Der erhaltene Wert entspricht der Sättigungslöslichkeit von Drospirenon in der eingetrockneten Prüflösung (Drospirenon$_{S2}$ [mg/ml])

*im Fall, dass weniger als 1 Gramm entnommen werden können, typischerweise bei $F_E$ >200, kann der Ansatz im Rotavapor entsprechend vergrößert werden oder die abschließende Bestimmung wird beispielsweise auf 50 mg Drospirenon in 0,5 g eingetrockneter Prüflösung reduziert.

Der Übersättigungsfaktor $F_{SS}$ bezogen auf Drospirenon in der Prüflösung während der Eintrocknung wird nach Gleichung 3 errechnet, wobei die Initialkonzentration von Drospirenon in der Zubereitung als Dospirenon$_{Init}$ [mg/ml] eingeht:

$$F_{SS} = Drospirenon_{Init} \ [mg/ml] \ x \ F_E \ / \ Drospirenon_{S2} \ [mg/ml]$$

Auf den so ermittelten Übersättigungsfaktor $F_{SS}$ wird in den Ansprüchen dieser Patentanmeldung Bezug genommen.

Ein Wert für $F_{SS}$ von 5 bedeutet beispielsweise, dass die Sättigungslöslichkeit von Drospirenon während der Anwendung der Zubereitung um den Faktor 5 überschritten wird.

- Bei transdermalen Pflastern vom Reservoirtyp:

Es wird die Löslichkeit von Drospirenon in der Formulierung des Wirkstoffreservoirs betrachtet.

Es gilt wiederum der halbtheoretische Ansatz, dass eine Prüflösung allein aus den flüssigen Bestandteilen stellvertretend für die halbfeste Reservoirformulierung untersucht wird.

Die Ermittlung der Sättigungslöslichkeit in der Reservoirzubereitung wird auf dieser Grundlage analog zu der Bestimmung von Drospirenon$_{S1}$ [mg/ml] in Gelen, Lotionen und Sprays, wie oben, durchgeführt.

Der Eintrocknungsfaktors $F_E$ tritt hier für die Bestimmung des Konzentrierungsfaktors $F_C$, da das Reservoir durch transdermale Permeation von flüssigen Bestandteilen eintrocknet.

Die Wirkstoff abgebende Fläche des Reservoirsystems wird mit dieser Folie abgedeckt, und das Reservoirsystem wird in dieser Anordnung gewogen ($G_{RS1}$). Danach wird das Reservoirsystem über den vorgesehenen Zeitraum bei einer Temperatur von 32°C reinem Wasser als Akzeptorflüssigkeit ausgesetzt. Der vorgesehene Zeiraum beträgt bei einem System zur einmal täglichen Applikation 24 Stunden.

Danach wird das System gewogen ($G_{RS2}$),

Aus dem ermittelten Gewichtsverlust ergibt sich der Eintrocknungssfaktor $F_E$ in der Reservoirzubereitung nach Gleichung 4:

$$F_E = G_{RF} / (G_{RF}-(G_{RS1} - G_{RS2})),$$

wobei $G_{RF}$ das Gewicht der vor dem Versuch in dem Reservoirsystem enthaltenen Menge an wirkstoffhaltiger Reservoirformulierung ist.

Die Ermittlung der Sättigungslöslichkeit von Drospirenon in der aufkonzentrierten Reservoirformulierung ($Drospirenon_{S2}$) und der Übersättigungsfaktor $F_{SS}$ werden analog den Ausführungen unter Gelen, Lotionen und Sprays ab Gleichung 1 ausgeführt, wobei $F_C$ an die Stelle von $F_E$ tritt.

**Ausführungsbeispiele**

Biespiel 1 - Hydro-ethanolisches Gel

|  | Teile (m/m) |
|---|---|
| Drospirenon | 1,0 |
| Testogel® | 99,0 |

Drospirenon wird direkt mit dem kommerziell erhältlichen Testogel vermischt und in diesem vollständig aufgelöst.

Testogel entspricht in seiner Zusammensetzung dem Androgel®:

| 100 g Gel enthalten | Testosteron | 1.0 g |
|---|---|---|
|  | Carbopol 980 | 0.90 g |
|  | Isopropyl myristat | 0.50 g |
|  | 0.1 N NaOH | 4.72 g |
|  | Ethanol (95% w/w) | 72.5 g* |
|  | gereinigtes Wasser | zu 100 g |
|  | *entsprechend 67 g | Ethanol. |

Beispiel 2 - Hydro-ethanolisches Gel auf Polyacrylat-Basis

|  | Teile (m/m) |
|---|---|
| Drospirenon | 1,0 |
| Ethinylestradiol | 0,1 |
| Isopropylmyristat | 1,0 |
| Carbopol 940 | 0,7 |
| 0.1 N NaOH | 3,7 |
| gereinigtes Wasser | 20 |
| Ethanol 96% | zu 100,0 |

Die Herstellung erfolgt in der dem Fachmann bekannten Weise durch Auflösen/Mischen aller Komponenten und abschließender Neutralisation des Gelbildners mit verdünnter Natronlauge, wobei die Gelbildung einsetzt. Isoporpylmyristat dient als Rückfettungsmittel auf der Haut.

Die Übersättigung an Drospirenon tritt während der Applikation durch die Verdunstung von Ethanol ein.

Beispiel 3 - Hydro-ethanolisches Gel auf Cellulose-Basis mit kristallisationshemmendem Zusatz

|                      | Teile (m/m) |
|----------------------|-------------|
| Drospirenon          | 0,5         |
| Ethyloleat           | 2,0         |
| Kollidon 12 PF       | 0,5         |
| Hydroxyethylcellulose| 1,0         |
| Wasser               | 30,0        |
| Ethanol 96%          | zu 100,0    |

Die Herstellung erfolgt in der dem Fachmann bekannten Weise durch Ansatz des Gelbildner Methylcellulose mit Wasser und Alkohol und abschließender Zugabe der Hilfs- und Wirkstoffe.

Ethyloleat dient als Permeationsbeschleuniger.

Die Übersättigung an Drospirenon tritt während der Applikation durch die Verdunstung von Ethanol ein.

Beispiel 4 - Transdermales Pflaster vom Typ eines Reservoirsystems

| Drospirenon            | 5,0 mg*  |
|------------------------|----------|
| Ethanol 96% etwa       | 196 $\mu$l |
| Androderm 2,5 mg Pflaster | 1 Stück |

Von einem zuvor genau gewogenen Androderm Pflaster wird die Schutzhülle abgezogen und es wird mit der nun offen liegenden Membran nach oben liegend der Inhalt unter Raumbedingungen verdunsten gelassen, bis etwa 180 mg Inhalt verdunstet sind. Danach wird das Reservoir wieder mit der Schutzfolie abgedeckt. Diese Vorbehandlung ist erforderlich, um in dem Reservoir genügend Platz für die nachfolgend beschriebene Zugabe von Drospirenon-Lösung zu schaffen.

Es werden 100 mg Drospirenon in 4 ml Ethanol 96% unter Einwirkung von Ultraschall gelöst. Von dieser Lösung werden etwa 500 $\mu$l in eine 1 ml Einmalspritze mit angesetzter Stahlkanüle (0,9 x 40 mm) aufgezogen. Mit dieser Spritze wird sodann in das Reservoir des Androdermpflasters eingestochen und etwa 200 $\mu$l Lösung werden in das Reservoir injiziert. Dies wird durch Einstich eines zusätzlichen Entlüftungsloches an der Stelle ermöglicht, an der sich die Luftblase in dem halbfesten Reservoir befindet.

Die beiden Einstichstellen werden danach mit einem Stück tesa-Film verklebt. Der Inhalt des Reservoirs wird durch vorsichtiges Kneten mit den Fingern einige Minuten lang vermischt. Bis zur experimentellen Verwendung wird das System mindestens 24 Stunden unter Raumbedingungen equilibriert.

Die Übersättigung an Drospirenon tritt während der Applikation des Systems im wesentlichen durch die transdermale Resorption von Ethanol ein, die wesentlich schneller verläuft, als die von Drospirenon.

Beispiel 5 - Transdermales Spray

|                  | Teile (m/m) |
|------------------|-------------|
| Drospirenon      | 0,5         |
| Ethinylestradiol | 0,1         |
| Oleylalkohol     | 1,0         |
| 1,2-Propandiol   | 0,5         |
| Ethanol 96%      | 60,0        |
| Dimethylether    | zu 100,0    |

Die Übersättigung an Drospirenon tritt während der Applikation im Wesentlichen durch die Verdunstung von Ethanol ein. Dimethylether dient als Treibmittel für das Spray.

Beispiel 6

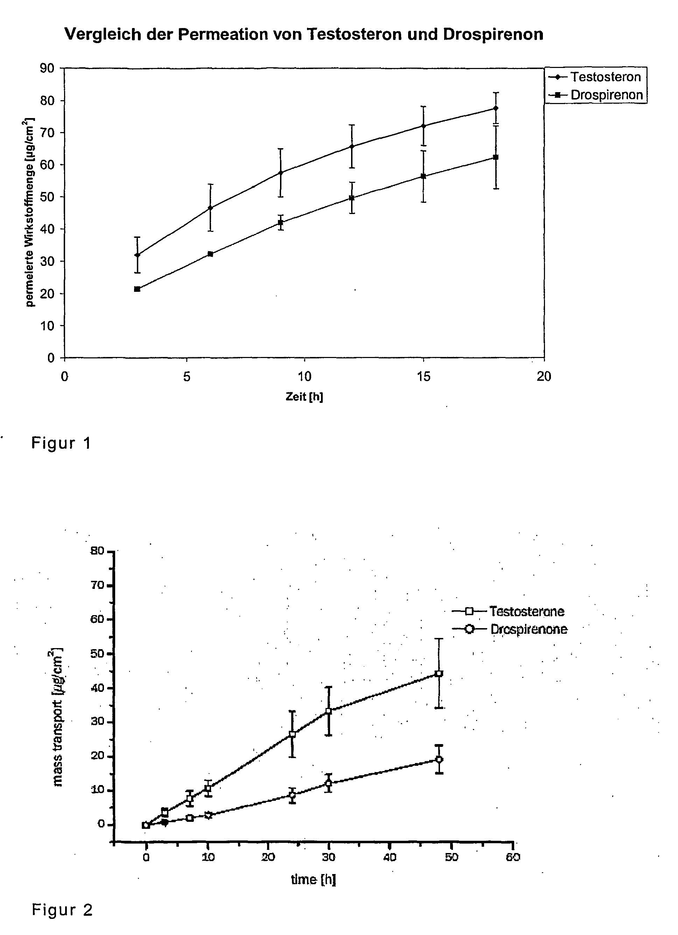

Die Formulierung aus Beispiel 1 wurde auf hinsichtlich der Permeation von Drospirenon im Vergleich zu Testosteron durch Maushaut untersucht - siehe Figur 1.

Von der Zubereitung aus Beispiel 1 wurden etwa 200 mg auf Nacktmaushaut appliziert, die als Permeationsmembran in modifizierten Franz-Permeationszellen fixiert war.

Die Resultate in Figur 1 sind die Mittelwerte von n=7 Experimenten. Haut: Haarlose Maushaut, HsdCpb NMRInu/nu, Harlan Bioservice, Walsrode

Akzeptormedium für die Franz-Zellen:

| | |
|---|---|
| Kaliumchlorid | 0,6 g |
| Kaliumdihydrogenphosphat | 0,09 g |
| Natriumchlorid | 10,905 g |
| Natriumhydrogenphosphatdihydrat | 0,09 g |
| HEPES | 8,94 g |
| Gentamicinsulfat | 0,075 g |
| Y-Cyclodextrin | 7,5 g |
| Aqua purificata | ad 1500 g |

Zu den Zeitpunkten 3, 6, 9, 12, 15 und 18 Stunden wurden Proben von je 100 $\mu$l durch ein automatisches Probenahmesystem aus dem Akzeptormedium der Franz-Zellen entnommen und in einen HPLC-Automaten injiziert.

| HPLC-Methode: | |
|---|---|
| Säule | LiChrospher 100 RP 18, 5 $\mu$m, 125 x 3 mm |
| Mobile Phase | Acetonitril/H2O (40/60) |
| Flussrate | 1 ml/min |
| Temperatur | 40°C |
| Injektionsvol. | 100 $\mu$l |
| Detektions-Wellenlänge (UV): | 244 nm (Testosteron), 261 nm (Drospirenon) |

Beispiel 7:

Die Formulierung aus Beispiel 1 wurde auf hinsichtlich der Permeation von Drospirenon im Vergleich zu Testosteron auf Humanhaut (weibliche Bauchhaut aus kosmetischer Reduktion, dermatomisiert auf ca. 450 $\mu$m Schichtdicke) untersucht - siehe Figur 2.

Tab. 1: Permeation von Testosteron aus Beispiel 1 durch Humanhaut: Durchschnittliche kumulative Permeation [$\mu$g/cm$^2$] von 1 % Testosteron in Testogel (K2053) durch dermatomisierte Humanhaut

| Zeit [h]) | Durchschnitt | SD | RSD [%] |
|---|---|---|---|
| 0 | 0.00 | 0.00 | - |
| 3 | 3.62 | 1.24 | 34 |
| 7 | 7.72 | 2.21 | 29 |
| 10 | 10.70 | 2.34 | 22 |
| 24 | 26.57 | 6.72 | 25 |
| 30 | 33.31 | 7.06 | 21 |
| 48 | 44.35 | 10.03 | 23 |

Tab. 2: Permeation von Drospirenon aus Beispiel 1 durch Humanhaut Durchschnittliche kumulative Permeation [$\mu$g/cm$^2$] von 1 % Drospirenon in Testogel (K2053) durch dermatomisierte Humanhaut

| Zeit [h]) | Durchschnitt | SD | RSD [%] |
|---|---|---|---|
| 0 | 0.00 | 0.00 | - |
| 3 | 0.87 | 0.34 | 39 |
| 7 | 2.04 | 0.68 | 33 |
| 10 | 2.86 | 0.75 | 26 |
| 24 | 8.67 | 2.19 | 25 |
| 30 | 12.13 | 2.64 | 22 |
| 48 | 19.23 | 4.03 | 21 |

Aus diesen Daten kann geschlossen werden, dass bei der Applikation auf Humanhaut die Permeation von Drospirenon noch bis zu 40% derjenigen von Testosteron beträgt. Nachdem Testogel für die Verabreichung von 5 bis 10 mg pro Tag zugelassen ist, kann geschlossen werden, dass auch etwa 3-5 mg/d Drospirenon in dieser Form transdermal verabreicht werden können. Damit wird die transdermale Applikation kontrazeptiv wirkender Mengen von Drospirenon ermöglicht.

**Patentansprüche**

1.  Pharmazeutische Zubereitung zur Applikation auf die Haut mit einem Gehalt an Drospirenon,
    **dadurch gekennzeichnet,**
    **dass** die enthaltene Menge Drospirenon in der Zubereitung in deren Ausgangszustand nicht oberhalb der Sättigungslöslichkeit liegt, während die Sättigungslöslichkeit des Drospirenons nach dem Auftragen der Zubereitung auf die Haut durch Austritt von lösungsvermittelnden Komponenten aus der Zubereitung überschritten wird.

2.  Pharmazeutische Zubereitung nach Anspruch 1,
    **dadurch gekennzeichnet, dass** die Sättigungslöslichkeit des Drospirenons in der Zubereitung während der Applikation um mindestens den Faktor 5, vorzugsweise um mindestens den Faktor 10 überschritten wird.

3.  Pharmazeutische Zubereitung nach Anspruch 2,
    **dadurch gekennzeichnet, dass** die Sättigungslöslichkeit des Drospirenons in der Zubereitung während der Applikation nach 1 Stunde bereits um mindestens den Faktor 2, vorzugsweise um mindestens den Faktor 5 überschritten wird.

4.  Pharmazeutische Zubereitung nach einem oder mehreren der vorangehenden Ansprüche,
    **dadurch gekennzeichnet, dass** sie einen Gehalt an Ethanol oder Isopropanol aufweist.

5.  Pharmazeutische Zubereitung nach einem oder mehreren der vorangehenden Ansprüche,
    **dadurch gekennzeichnet, dass** bezogen auf Drospirenon ein kristallisationshemmender Zusatz enthalten ist.

6.  Pharmazeutische Zubereitung nach einem oder mehreren der vorangehenden Ansprüche,
    **dadurch gekennzeichnet, dass** es sich bei dem kristallisationshemmenden Zusatz um ein lösliches Polyvinylpyrrolidon handelt.

7.  Pharmazeutische Zubereitung nach einem oder mehreren der vorangehenden Ansprüche, **dadurch gekennzeichnet,**
    **dass** es sie einen Gehalt an mindestens einem Permeationsbeschleuniger aufweist.

8.  Pharmazeutische Zubereitung nach einem oder mehreren der vorangehenden Ansprüche,
    **dadurch gekennzeichnet, dass** es sich um eine halbfeste Zubereitung in der Form eines alkoholhaltigen Gels handelt .

9.  Pharmazeutische Zubereitung nach einem oder mehreren der vorangehenden Ansprüche,

**dadurch gekennzeichnet, dass** es sich um eine flüssige, alkoholhaltige Zubereitung in der Form einer Lotion, eines Schaums oder eines Sprays handelt.

10. Pharmazeutische Zubereitung nach einem oder mehreren der vorangehenden Ansprüche, **dadurch gekennzeichnet, dass** es sich um eine flüssige oder halbfeste, alkoholhaltige Zubereitung als Bestandteil eines transdermalen Pflasters vom Typ eines Reservoirsystems handelt.

11. Pharmazeutische Zubereitung nach Anspruch 10, **dadurch gekennzeichnet, dass** die Sättigungslöslichkeit des Drospirenons in der Zubereitung während der Applikation um mindestens den Faktor 1,5 , vorzugsweise um mindestens den Faktor 2 überschritten wird.

12. Pharmazeutische Zubereitung nach einem der Ansprüche 9 bis 11, **dadurch gekennzeichnet, dass** die alkoholische Zubereitung einen Alkoholgehalt von mindestens 60% (m/m), vorzugsweise von mindestens 65% (m/m) aufweist.

13. Pharmazeutische Zubereitung nach einem oder mehreren der vorangehenden Ansprüche, **dadurch gekennzeichnet, dass** zusätzlich zu Drospirenon ein Estrogen enthalten ist.

14. Pharmazeutische Zubereitung nach Anspruch 13, **dadurch gekennzeichnet, dass** das enthaltene Estrogen während der Applikation auf der Haut ebenfalls vom untersättigten oder gerade gesättigten Zustand in den übersättigten Zustand übergeht.

15. Pharmazeutische Zubereitung nach Anspruch 13 oder 14, **gekennzeichnet dadurch, dass** es sich bei dem Estrogen um Ethinylestradiol handelt.


**Claims**

1. Pharmaceutical preparation for application to the skin having a content of drospirenone, **characterized in that** the amount of drospirenone contained in the preparation in the initial state thereof is not above the saturation solubility, while the saturation solubility of drospirenone is exceeded after application of the preparation to the skin through efflux of solubilizing components from the preparation.

2. Pharmaceutical preparation according to claim 1, **characterized in that** the saturation solubility of drospirenone in the preparation during application is exceeded by a factor of at least 5, preferably by a factor of at least 10.

3. Pharmaceutical preparation according to claim 2, **characterized in that** the saturation solubility of drospirenone in the preparation during application is exceeded by a factor of at least 2, preferably by a factor of at least 5, after only 1 hour.

4. Pharmaceutical preparation according to one or more of the preceding claims, **characterized in that** it has a content of ethanol or isopropanol.

5. Pharmaceutical preparation according to one or more of the preceding claims, **characterized in that** a crystallization-inhibiting addition in relation to drospirenone is present.

6. Pharmaceutical preparation according to one or more of the preceding claims, **characterized in that** the crystallization-inhibiting addition is a soluble polyvinylpyrrolidone.

7. Pharmaceutical preparation according to one or more of the preceding claims, **characterized in that** it has a content of at least one permeation promoter.

8. Pharmaceutical preparation according to one or more of the preceding claims, **characterized in that** it is a semisolid preparation in the form of an alcohol-containing gel.

9. Pharmaceutical preparation according to one or more of the preceding claims, **characterized in that** it is a liquid, alcohol-containing preparation in the form of a lotion, of a foam or of a spray.

**10.** Pharmaceutical preparation according to one or more of the preceding claims, **characterized in that** it is a liquid or semisolid, alcohol-containing preparation as constituent of a transdermal patch of the reservoir system type.

**11.** Pharmaceutical preparation according to claim 10, **characterized in that** the saturation solubility of drospirenone in the preparation during application is exceeded by a factor of at least 1.5, preferably by a factor of at least 2.

**12.** Pharmaceutical preparation according to any of claims 9 to 11, **characterized in that** the alcoholic preparation has an alcohol content of at least 60% (m/m), preferably of at least 65% (m/m).

**13.** Pharmaceutical preparation according to one or more of the preceding claims, **characterized in that** an estrogen is present in addition to drospirenone.

**14.** Pharmaceutical preparation according to claim 13, **characterized in that** during application to the skin there is likewise a transition in the estrogen content from the subsaturated or just saturated state into the supersaturated state.

**15.** Pharmaceutical preparation according to claim 13 or 14, **characterized in that** the estrogen is ethinylestradiol.

**Revendications**

**1.** Préparation pharmaceutique pour l'application sur la peau avec une teneur en drospirénone, **caractérisée en ce que** la quantité de drospirénone contenue dans la préparation dans son état de départ n'est pas supérieure à la solubilité de saturation, alors que la solubilité de saturation de la drospirénone après l'application de la préparation sur la peau est dépassée par l'évacuation de composants promoteurs de solubilisation de la préparation.

**2.** Préparation pharmaceutique selon la revendication 1, **caractérisée en ce que** la solubilité de saturation de la drospirénone dans la préparation est dépassée pendant l'application d'au moins un facteur 5, de préférence d'au moins un facteur 10.

**3.** Préparation pharmaceutique selon la revendication 2, **caractérisée en ce que** la solubilité de saturation de la drospirénone dans la préparation est dépassée pendant l'application après 1 heure déjà d'au moins un facteur 2, de préférence d'au moins un facteur 5.

**4.** Préparation pharmaceutique selon l'une ou plusieurs des revendications précédentes, **caractérisée en ce qu'**elle présente une teneur en éthanol ou en isopropanol.

**5.** Préparation pharmaceutique selon l'une ou plusieurs des revendications précédentes, **caractérisée en ce qu'**un additif inhibiteur de la cristallisation par rapport à la drospirénone est contenu.

**6.** Préparation pharmaceutique selon l'une ou plusieurs des revendications précédentes, **caractérisée en ce qu'**il s'agit pour l'additif inhibiteur de la cristallisation d'une polyvinylpyrrolidone soluble.

**7.** Préparation pharmaceutique selon l'une ou plusieurs des revendications précédentes, **caractérisée en ce qu'**elle présente une teneur en au moins un accélérateur de la perméation.

**8.** Préparation pharmaceutique selon l'une ou plusieurs des revendications précédentes, **caractérisée en ce qu'**il s'agit d'une préparation semi-solide sous forme d'un gel contenant un alcool.

**9.** Préparation pharmaceutique selon l'une ou plusieurs des revendications précédentes, **caractérisée en ce qu'**il s'agit d'une préparation liquide contenant un alcool sous forme d'une lotion, d'une mousse ou d'un spray.

**10.** Préparation pharmaceutique selon l'une ou plusieurs des revendications précédentes, **caractérisée en ce qu'**il s'agit d'une préparation liquide ou semi-solide, contenant un alcool comme constituant d'un pansement transdermique du type d'un système à réservoir.

**11.** Préparation pharmaceutique selon la revendication 10, **caractérisée en ce que** la solubilité de saturation de la drospirénone dans la préparation est dépassée pendant l'application d'au moins un facteur 1,5, de préférence d'au moins un facteur 2.

**12.** Préparation pharmaceutique selon l'une quelconque des revendications 9 à 11, **caractérisée en ce que** la préparation alcoolique présente une teneur en alcool d'au moins 60% (m/m), de préférence d'au moins 65% (m/m).

**13.** Préparation pharmaceutique selon l'une ou plusieurs des revendications précédentes, **caractérisée en ce qu'**un estrogène est contenu en plus de la drospirénone.

**14.** Préparation pharmaceutique selon la revendication 13, **caractérisée en ce que** l'estrogène contenu pendant l'application sur la peau passe également d'un état inférieur à la saturation ou tout juste saturé à un état sursaturé.

**15.** Préparation pharmaceutique selon la revendication 13 ou 14, **caractérisée en ce qu'**il s'agit, pour l'estrogène, d'éthynylestradiol.

## Vergleich der Permeation von Testosteron und Drospirenon

Figur 1

Figur 2

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 5352457 A
- WO 9711680 A1
- DE 19906152 A1
- WO 9915156 A1